# FASCICULE DE BREVET EUROPEEN

(11) **EP 3 478 262 B1**
(45) Date de publication et mention de la délivrance du brevet: **13.11.2024**
(21) Numéro de dépôt: 17742802.6
(22) Date de dépôt: 20.06.2017
(51) Int. Cl.: A61K 8/92, A61K 36/28, A61Q 19/00, A61P 25/02, A61K 9/00, A61K 47/44

(54) **UTILISATION D'UNE HUILE ESSENTIELLE D'IMMORTELLE POUR AUGMENTER OU RESTAURER LA PERCEPTION TACTILE CUTANÉE**
VERWENDUNG EINES ÄTHERISCHEN ÖLS EINER IMMORTELLE ZUR ERHÖHUNG ODER WIEDERHERSTELLUNG EINER TAKTILEN WAHRNEHMUNG
USE OF AN ESSENTIAL OIL OF EVERLASTING FLOWER IN ORDER TO INCREASE OR RESTORE TACTILE PERCEPTION

(30) Priorité: 01.07.2016 FR 1656292
(43) Date de publication de la demande: 08.05.2019
(73) Titulaire: LABORATOIRES M & L, 04100 Manosque (FR)
(72) Inventeur: CENIZO, Valérie, 13650 Meyragues (FR); LEMAIRE, Géraldine, 04210 Valensole (FR); PORTES, Pascal, 13540 Puyricard (FR)
(74) Mandataire: Renard, Emmanuelle
(86) Numéro de dépôt international: PCT/FR2017/051610
(87) Numéro de publication internationale: WO 2018/002475

(56) Documents cités:
- EP-A1- 2 532 355
- OMIDREZA FIRUZI ET AL: "Modulation of neurotrophic signaling pathways by polyphenols", DRUG DESIGN, DEVELOPMENT AND THERAPY, 1 December 2015 (2015-12-01), pages 23, XP055320086, DOI: 10.2147/DDDT.S96936
- SUZGEC S ET AL: "Flavonoids of Helichrysum compactum and their antioxidant and antibacterial activity", FITOTERAPIA, IDB HOLDING, MILAN, IT, vol. 76, no. 2, 1 March 2005 (2005-03-01), pages 269 - 272, XP027752380, ISSN: 0367-326X, [retrieved on 20050301]
- SANDEEP VASANT MORE ET AL: "The Role of Bioactive Compounds on the Promotion of Neurite Outgrowth", MOLECULES ONLINE, vol. 17, no. 12, 4 December 2012 (2012-12-04), DE, pages 6728 - 6753, XP055320078, ISSN: 1433-1373, DOI: 10.3390/molecules17066728
- DANIEL ANTUNES VIEGAS ET AL: "Helichrysum italicum: From traditional use to scientific data", JOURNAL OF ETHNOPHARMACOLOGY, vol. 151, no. 1, 1 January 2014 (2014-01-01), IE, pages 54 - 65, XP055320080, ISSN: 0378-8741, DOI: 10.1016/j.jep.2013.11.005
- ANGE BIANCHINI ET AL: "Composition ofHelichrysum italicum (Roth) G. Don fil. subsp.italicum essential oils from Corsica (France)", FLAVOUR AND FRAGRANCE JOURNAL., vol. 16, no. 1, 1 January 2001 (2001-01-01), GB, pages 30 - 34, XP055228024, ISSN: 0882-5734, DOI: 10.1002/1099-1026(200101/02)16:1<30::AID-FFJ941>3.0.CO;2-F

## Description

### OBJET DE L'INVENTION

La présente invention concerne l'utilisation cosmétique non thérapeutique d'une huile essentielle d'immortelle de l'espèce *Helichrysum italicum,* appliquée topiquement sur la peau, pour lutter contre la diminution de la perception tactile cutanée due à l'âge. L'huile essentielle d'immortelle permet de développer les prolongements neuronaux des nerfs sensitifs cutanés humains.

### ARRIERE-PLAN DE L'INVENTION

La peau constitue un organe sensoriel majeur, dans la mesure où elle est en contact direct avec l'environnement extérieur. Elle reçoit continuellement une diversité de stimuli thermiques, mécaniques ou chimiques.

Plusieurs types de récepteurs sensitifs ont été identifiés dans la peau. Outre les thermorécepteurs, qui transmettent la sensibilité au chaud et au froid, les nocicepteurs, qui sont des récepteurs de la douleur, et les pruricepteurs, qui sont des récepteurs spécifiques du prurit, différents types de récepteurs mécaniques ont été identifiés. Ces derniers, qui transmettent la sensibilité à la pression et à la vibration et la sensibilité fine épicritique ou tact (qui répond à un contact léger avec la peau), sont constitués des terminaisons libres, des disques de Merkel localisés dans l'épiderme basal, des corpuscules de Meissner situés dans le derme papillaire, des corpuscules de Pacini localisés dans le derme profond et des corpuscules de Ruffini situés dans le derme. La répartition de ces récepteurs est différente dans la peau glabre et la peau poilue. Leur densité varie également selon les régions ; ainsi, la paume de la main est particulièrement bien fournie et la peau du visage renferme également de nombreux récepteurs.

Ces récepteurs mécaniques sont innervés par des neurones sensitifs appelés mécanorécepteurs à bas seuil (LTMR pour "low-threshold mechanoreceptors") qui réagissent aux stimulations mécaniques inoffensives. Ils sont classés en différents types, en fonction de la taille de leurs corps cellulaires, du diamètre des axones, du degré de myélinisation et de la vitesse de conduction axonale. Les neurones sensitifs fournissent des informations au système nerveux central qui génère en retour une réponse appropriée. Il est ainsi possible de reconnaître des objets et discriminer des textures, notamment.

Ces neurones sensitifs comprennent un corps cellulaire dont émergent des neurites (axone et dendrites). Les neurites sont généralement enveloppées ou en contact avec les récepteurs mentionnés précédemment, en particulier les cellules de Merkel.

Or, avec l'âge ou dans certaines pathologies, notamment les neuropathies périphériques dues par exemple au diabète, à la chimiothérapie ou à la consommation d'alcool, ou encore les maladies neurodégénératives telles que la sclérose en plaque, on observe une diminution de la perception tactile ou hypoesthésie. On considère ainsi que les déficits tactiles touchent plusieurs centaines de milliers de personnes en France. Néanmoins, leur prise en compte reste marginale.

On comprend donc qu'il serait utile de disposer d'un moyen permettant de restaurer la perception tactile cutanée ou de l'augmenter.

Il a déjà été suggéré que l'administration orale (EP 2 438 916 ; US 2002/040052) ou topique (KR 2013 0107982) de certains extraits de certains plantes pouvait conduire à un allongement des neurites. Cet effet n'a toutefois été mis à profit que pour traiter certaines maladies neurodégénératives ou en vue d'améliorer les capacités d'apprentissage, de concentration ou de mémorisation. Il a également été suggéré que certains polyphénols tels que le curcumin présentaient un effet neurotrophique (Omidreza Firuzi et al., Drug Design, Development and Therapy, 2015, 23).

La présente invention vise à proposer un autre extrait végétal permettant de favoriser l'allongement des neurites. Elle a également pour objectif de proposer un extrait végétal permettant de restaurer ou d'augmenter la perception tactile cutanée, notamment lors de l'application d'un produit cosmétique.

Il a été mis en évidence qu'un extrait d'immortelle présentait cet effet. Différents extraits d'immortelle, ainsi que leurs constituants, sont connus (Daniel Antunes Viegas et al., Journal of Ethnopharmacology, 2014, 151, n°1, 54-65) ; toutefois, il n'a encore jamais été suggéré qu'une huile essentielle d'immortelle permettait d'atteindre l'objectif précité.

### RESUME DE L'INVENTION

Dans ce contexte, l'invention a pour objet l'utilisation cosmétique non thérapeutique d'une huile essentielle d'immortelle de l'espèce *Helichrysum italicum,* appliquée topiquement sur la peau, pour lutter contre la diminution de la perception tactile cutanée due à l'âge.

Elle a encore pour objet un procédé cosmétique non thérapeutique pour lutter contre la diminution de la perception tactile cutanée due à l'âge, comprenant l'application topique sur la peau d'une huile essentielle d'immortelle de l'espèce *Helichrysum italicum.*

Elle a également pour obj et une huile essentielle d'immortelle de l' espèce *Helichrysum italicum,* pour son utilisation topique dans un traitement visant à augmenter ou à restaurer la perception tactile cutanée, par application sur la peau de personnes souffrant de neuropathies périphériques, de maladies neurodégénératives ou d'une destruction de terminaisons nerveuses consécutive à une plaie telle qu'une brûlure.

### DESCRIPTION DETAILLEE

La présente invention met en oeuvre une huile essentielle d'immortelle de l'espèce *Helichrysum italicum* (ou *Helichrysum angustifolium* D.C), qui est l'immortelle d'Italie, quelle que soit la sous-espèce considérée.

Par "huile essentielle", on entend dans cette description le produit d'hydrodistillation ou d'entraînement à la vapeur des composés organiques volatils présents dans une partie quelconque de l'immortelle ou de la plante entière, et plus particulièrement dans ses parties aériennes comme par exemple ses fleurs ou sommités fleuries.

L'huile essentielle d'immortelle peut être incluse dans une composition cosmétique et elle représente avantageusement dans ce cas de 0,001 à 5% en poids et préférentiellement de 0,01 à 2% en poids, par rapport au poids total de la composition utilisée selon l'invention.

Comme il ressort des exemples ci-après, il a été démontré que l'huile essentielle d'immortelle permet de favoriser la neuritogénèse, plus précisément de développer les prolongements neuronaux des nerfs sensitifs cutanés humains, de sorte qu'elle peut être utilisée pour restaurer ou augmenter la perception tactile cutanée. Cette propriété peut être mise à profit en utilisant l'huile essentielle d'immortelle en tant que telle, ou au sein d'une composition cosmétique à usage topique, sous un produit de soin de la peau. En variante, la composition cosmétique renfermant l'huile essentielle d'immortelle peut constituer elle-même un produit de soin de la peau. L'huile essentielle d'immortelle permet ainsi d'augmenter les sensations tactiles ressenties par la personne sur laquelle ce produit de soin est appliqué, lors de l'application du produit, et notamment de mieux percevoir sa texture ou son glissant. L'huile essentielle d'immortelle ou la composition la comprenant peut être appliquée sur toute partie du corps et de préférence sur la peau du visage et/ou des mains, qui sont les zones les plus innervées. Elle peut en particulier être appliquée sur la peau de personnes souffrant d'une perte de sensations tactiles, en particulier de personnes âgées (typiquement de femmes ménopausées) ou souffrant de pathologies, notamment de neuropathies périphériques, dues notamment au diabète ou à la chimiothérapie, de maladies neurodégénératives, telles que la sclérose en plaque, ou d'une destruction de terminaisons nerveuses consécutive à une plaie telle qu'une brûlure.

Cette composition renferme généralement un milieu physiologiquement acceptable, en particulier cosmétiquement acceptable, c'est-à-dire qui ne génère pas de picotements ou de rougeurs incompatibles avec une utilisation cosmétique. Ce milieu renferme de préférence une phase aqueuse en plus de la phase huileuse. On préfère que la composition se présente sous forme d'émulsion huile-dans-eau ou eau-dans-huile.

La phase aqueuse peut notamment contenir, outre de l'eau, au moins un constituant choisi parmi les gélifiants aqueux et les actifs hydrophiles tels que les humectants.

Par "gélifiant aqueux", on désigne un composé polymérique capable d'immobiliser des molécules d'eau en s'hydratant et d'augmenter ainsi la viscosité de la phase aqueuse. Un tel gélifiant peut être choisi parmi : les polysaccharides, tels que : la cellulose et ses dérivés, les amidons modifiés, le carraghénane, l'agar agar, la gomme de xanthane et les gommes végétales telles que la gomme de guar ou de caroube ; les polymères synthétiques et notamment les homopolymères d'acrylate de sodium avantageusement réticulés, ainsi que les copolymères acryliques, en particulier les copolymères d'acrylate de sodium et/ou de (méth)acrylate d'alkyle et/ou de (méth)acrylate d'hydroxyalkyle et/ou de (méth)acrylate de (polyéthoxy)alkyle, avec au moins un autre monomère, avantageusement l'acide 2-acrylamido-2-méthylpropane sulfonique (AMPS), ces copolymères étant de préférence réticulés; et leurs mélanges.

De son côté, la phase grasse peut comprendre, outre l'huile essentielle d'immortelle, une ou plusieurs huiles volatiles et/ou non volatiles. Des exemples d'huiles volatiles sont les alcanes ramifiés, tels que l'isododécane, et les alcanes linéaires en C₁₀-C₁₃. Comme huiles non volatiles, on peut citer notamment :
- les esters d'acides et de mono-alcool choisis parmi : les mono- et polyesters d'acides linéaires saturés en C2-C10 (de préférence en C6-C10) et de mono-alcools linéaires saturés en C10-C18 (de préférence C10-C14), les mono- et polyesters d'acides linéaires saturés en C10-C20 et de mono-alcools ramifiés ou insaturés en C3-C20 (de préférence C3-C10) ; les mono- et polyesters d'acides ramifiés ou insaturés en C5-C20 et de mono-alcools ramifiés ou insaturés en C5-C20 ; les mono- et polyesters d'acides ramifiés ou insaturés en C5-C20 et de mono-alcools linéaires en C2-C4 ;
- les triglycérides d'acides gras en C6-C12, tels que les triglycérides d'acides caprylique et caprique et la triheptanoïne ;
- les acides gras ramifiés et/ou insaturés en C10-C20 (tels que les acides linoléique, laurique et myristique) ;
- les alcools gras ramifiés et/ou insaturés en C10-C20 (tels que l'octyldodécanol et l'alcool oléylique) ;
- les hydrocarbures tels que le squalane (C30), notamment le squalane végétal extrait de l'huile d'olive, et l'hémisqualane (C15) ;
- les carbonates de dialkyle, tels que le dicaprylyl carbonate et le diéthylhexyl carbonate ;
- les dialkyléthers tels que le dicaprylyl éther ; et
- leurs mélanges.

On peut également citer les huiles végétales qui contiennent un ou plusieurs des constituants précités.

Comme esters d'acides et de monoalcools, on peut notamment citer les monoesters tels que le mélange de caprate et caprylate de coco, le macadamiate d'éthyle, l'ester éthylique de beurre de karité, l'isostéarate d'isostéaryle, l'isononanoate d'isononyle, l'isononanoate d'éthylhexyle, le néopentanoate d'hexyle, le néopentanoate d'éthylhexyle, le néopentanoate d'isostéaryle, le néopentanoate d'isodécyle, le myristate d'isopropyle, le myristate d'octyldodécyle, le palmitate d'isopropyle, le palmitate d'éthylhexyle, le laurate d'hexyle, le laurate d'isoamyle, le nonanoate de cétostéaryle, le capylate de propylheptyle et leurs mélanges. D'autres esters utilisables sont les diesters d'acides et de monoalcools tels que l'adipate de disopropyle, l'adipate de diéthylhexyle, le sébaçate de diisopropyle et le sébaçate de diisoamyle.

Des exemples d'huiles végétales sont notamment les huiles de germe de blé, de tournesol, d'argan, d'hibiscus, de coriandre, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de cassis, d'onagre, de lavande, de bourrache, de millet, d'orge, de quinoa, de seigle, de carthame, de bancoulier, de passiflore, de rosier muscat, d'Echium, de cameline ou de camélia.

La phase grasse peut en outre comprendre au moins un structurant de phase grasse.

Par "structurant de phase grasse", on entend un composé capable d'épaissir les huiles contenues dans la composition, choisi notamment parmi les cires, les gélifiants de phase grasse et les corps gras pâteux, ainsi que leurs mélanges.

Le terme "cire" désigne, dans le contexte de cette description, un corps gras solide à 25°C, à changement d'état solide / liquide réversible, ayant un point de fusion généralement compris entre 30 et 160°C, de préférence entre 50 et 90°C, tel que mesuré par DSC. Des exemples de cires sont en particulier les cires d'origine animale ou végétale, telles que les cires d'abeille, de candelilla, de carnauba ou d'acacia ; les huiles végétales hydrogénées et éventuellement modifiées par l'acide isostéarique, notamment les huiles de colza, de soja, de tournesol, de jojoba, de coprah et de ricin hydrogénées ; les esters d'acides gras linéaires saturés en C₁₄-C₃₀ et d'alcools gras linéaires saturés en C₁₆-C₃₆ ; les acides linéaires et saturés en C₁₀-C₃₀ ; les alcools linéaires et saturés en C₈-C₃₀ ; et leurs mélanges. Ces cires peuvent se trouver sous forme micronisée, c'est-à-dire sous la forme d'une poudre dont les particules présentent une taille moyenne en nombre inférieure ou égale à 50 µm, et notamment allant de 0,5 à 50 µm, de préférence allant de 1 à 30 µm, voire allant de 3 à 20 µm, où la « taille moyenne en nombre » correspond à la dimension donnée par la distribution granulométrique statistique à la moitié de la population, dite D50.

Par "gélifiants de phase grasse", il est fait référence aux composés modifiant la rhéologie de la phase grasse par formation d'un réseau tridimensionnel. Comme composés de ce type, on peut notamment citer les argiles (en particulier les bentonites et les hectorites) modifiées hydrophobes, notamment par du chlorure de di-stéaryl diméthyl ammonium ; les silices pyrogénées modifiées hydrophobes ; le palmitate et le myristate de dextrine ; les polyamides, les copolymères oléfine(s) / styrène, les poly(acrylates d'alkyle) ; les glycérides d'acides gras (de préférence linéaires et saturés) en C₁₆-C₂₆ tels que le composé Nomcort^{®} HKG ; les dérivés de cellulose et les mélanges en contenant ; et leurs mélanges. Certaines huiles végétales hydrogénées peuvent également être considérées comme des gélifiants de phase grasse.

Enfin, les corps gras pâteux utilisables comme structurants de phase grasse sont définis comme des corps gras à changement d'état liquide / solide réversible, présentant à l'état solide une organisation cristalline anisotrope et comportant à une température de 23°C une fraction liquide et une fraction solide. On préfère utiliser les beurres végétaux. Les beurres de karité, de cacao et de mangue constituent des exemples de tels corps gras pâteux.

La composition utilisée selon l'invention peut par ailleurs comprendre un ou plusieurs émulsionnants, parfums, conservateurs, séquestrants, anti-oxydants, ajusteurs de pH, filtres UV, charges pulvérulentes, pigments, colorants et leurs mélanges.

Selon une forme d'exécution préférée, cette composition renferme en outre au moins un actif anti-âge, en particulier un actif adapté à prévenir et/ou traiter les rides, le relâchement cutané et/ou la formation de taches pigmentaires, qui peut notamment être choisi parmi les agents anti-radicalaires, les agents stimulant la différenciation et/ou la prolifération des kératinocytes et/ou des fibroblastes ; les agents stimulant la synthèse de glycosaminoglycanes et/ou de collagène et/ou de fibrilles d'ancrage dermo-épidermique ; les agents prévenant la dégradation du collagène et/ou des glycosaminoglycanes et/ou des fibrilles d'ancrage dermo-épidermique ; les agents antiglycation ; les agents dépigmentants et/ou inhibant la mélanogénèse ; et leurs mélanges. En variante et/ou en plus, la composition selon l'invention peut comprendre au moins un actif à effet neuronal tel que des agents apaisants et/ou inhibant l'inflammation neurogène ; les agents prévenant le neuro-vieillissement ; les agents modulant la libération d'acétylcholine par les neurones ; les agents réduisant l'exocytose neuronale ; et leurs mélanges.

Des exemples de tels actifs anti-âge et/ou à effet neuronal sont notamment : l'acide ascorbique, ses sels, ses éthers et ses esters, notamment le glucoside d'ascorbyle ; l'adénosine ; le ribose ; les extraits de miel ; les protéines et glycoprotéines, extraites notamment d'amande douce ; les protéines végétales hydrolysées, notamment issues du riz, des graines d'hibiscus ou du lupin ; les polypeptides et les pseudodipeptides, tels que le chlorhydrate de carcinine, le palmitoyl pentapeptide-4 (Pal-Lys-Thr-Thr-Lys-Ser) et le palmitoyl tripeptide-38 commercialisés notamment par SEDERMA sous les dénominations commerciale Matrixyl^{®} 3000 et Matrixyl^{®} Synthe'6, respectivement, le palmitoyl tripeptide-8 commercialisé par la société LUCAS MEYER sous la référence commerciale Nutrazen^{®}, le pentapeptide-18 commercialisé par la société LIPOTEC sous la dénomination commerciale Leuphasyl^{®} Solution, le sh-decapeptide-9 commercialisé par la société SANDREAM sous la dénomination commerciale Neoendorphin^{®} et le palmitoyl hexapeptide-52 commercialisé par la société INFINITEC sous la référence commerciale X50 Myocept^{®} Powder ; les silanes tels que le mannuronate de méthylsilanol ; les arabinoxylanes, extraits en particulier de farine de seigle et les galactoarabinanes, issus notamment du mélèze ; l'acide hyaluronique et ses sels ; les polyphénols, extraits en particulier de mimosa ; les alphahydroxyacides, dont ceux extraits de citron; les extraits aqueux de plantes telles que le trèfle d'eau, la pensée sauvage, la prêle des champs, le chardon aux ânes (*Onopordum acanthium*), le millefeuille (*Achillea millefolium,* contenu notamment dans le produit Neurobiox^{®} de la société BASF), l'embelia (*Embelia concinna,* telle que commercialisée par la société SEPPIC), le figuier de Barbarie (*Opuntia ficus indica,* commercialisé notamment par MIBELLE AG BIOCHEMISTRY sous la dénomination commerciale AquaCacteen^{®}), la sauge (*Salvia officinalis,* vendue notamment par PROVITAL GROUP), Vitex negundo (commercialisé notamment par les LABORATOIRES EXPANSCIENCE sous la référence commerciale Neurovity^{®}), la châtaigne, la papaye, l'arganier, l'avoine, le tournesol, la pâquerette, la pivoine ou l'aneth ; les extraits aqueux d'algues et notamment de coralline, de janie rouge, d'*Ungaria pinnatifada,* d'*Alaria esculenta* ou de *Nannochlorosis oculata* ; les huiles essentielles, notamment de myrte ; les gluconates de zinc et/ou de cuivre ; et leurs mélanges.

En variante ou en plus, la composition utilisée selon l'invention peut comprendre au moins un polymère tenseur, c'est-à-dire capable de tendre la peau par action mécanique et de réduire ainsi l'apparence des rides et des ridules. Il peut s'agir d'un polymère synthétique ou naturel, en particulier d'un polysaccharide, notamment d'un extrait d'algue ou de plancton marin ou d'une gomme végétale.

Cette composition peut se présenter sous toute forme adaptée à une application topique sur la peau et notamment sous forme de lait, de crème, de lotion, de gel ou de masque. Il s'agit généralement d'une composition non rincée.

### EXEMPLES

L'invention sera mieux comprise à la lumière des exemples suivants, qui sont donnés à titre purement illustratif et n'ont pas pour but de limiter la portée de l'invention, définie par les revendications annexées.

### Exemple 1 : Test in vitro sur co-culture de neurones sensitifs et kératinocytes humains

### 1. Matériel et méthodes

### 1.1. Culture et différenciation des hiPS

Des neurones sensitifs sont dérivés de cellules iPS (*induced Pluripotent Stem cells*) elles-mêmes obtenues à partir de fibroblastes humains. Les iPS ont été ensemencées en plaques 6 puits revêtues par une fine couche de Matrigel^{®} (Corning) à raison de 250 000 cellules par puits dans un milieu de différenciation constitué de DMEM- F12 (Panbiotech) supplémenté par 10% de Knockout Serum Replacement (KSR, Life technologies), 5µ M de CHIR 99021 (Tocris), 0,1 µM d'acide rétinoïque (Sigma), 1 µg/mL d'EPO (*Erythropoïétine* ; Peprotech), un cocktail d'inhibiteurs des voies de différenciation centrales et 1% d'antibiotiques PS (*Penicilin-Streptomycin* ; Panbiotech). Les cellules ont été maintenues 6 jours en culture à 37°C et 5% de CO2. Le milieu de culture a été changé tous les 2 jours. Dans ces conditions de culture, les iPS se différencient en neurones sensitifs humains.

A ce stade, les cellules ont été dissociées à l'aide d'accutase (Sigma Aldrich) pendant 10 minutes à 37°C. La réaction a été stoppée par ajout de milieu de culture. La suspension cellulaire a été centrifugée 5 minutes à 1200 tours/min. La viabilité cellulaire a été établie par comptage cellulaire au bleu de trypan et les cellules ont été ensemencées en plaques 96 puits revêtues par une fine couche de Matrigel^{®} à raison de 20 000 cellules par puits en milieu de différenciation.

Des kératinocytes issus d'un donneur adulte (Lonza) ont été préalablement amplifiés, en milieu de croissance pour kératinocytes (Lonza), sur un cycle, avant d'être dissociés par trypsination et congelés. Ces kératinocytes ont été décongelés et amplifiés à nouveau sur un cycle en milieu de croissance pour kératinocytes.

Après 24 heures d'adhésion des neurones en plaques 96 puits, les kératinocytes ont été dissociés par trypsination. La viabilité cellulaire a été établie par comptage cellulaire et les kératinocytes ont été ensemencés au-dessus des neurones à raison de 30 000 cellules par puits dans un milieu de culture constitué de 2/3 de milieu de maturation pour les neurones sensitifs et 1/3 de milieu de croissance pour kératinocytes. Le milieu de maturation était composé de DMEM-F12 supplémenté par 1% de N2 (Life technologies), 1% de PS, 10 ng/mL de NGF (*Nerve Growth Factor* ; Sigma Aldrich), 10 ng/mL de BDNF (*Brain-Derived Neurotrophic Factor* ; PanBiotech), 10ng/mL de NT3 (*NeuroTrophin-3* ; PanBiotech) et 10ng/mL de GDNF (*Glial Derived Neurotrophic Factor* ; PanBiotech).

### 1.2. Cytotoxicité de l'huile essentielle d'immortelle

Dans une première culture, après 24 heures de co-culture, le milieu a été changé par du milieu constitué de 2/3 de milieu de maturation des neurones sensitifs humains sans facteur de croissance et 1/3 de milieu de croissance pour kératinocytes, en présence ou non du composé à tester à différentes concentrations allant de 0,001% à 0,01%. Pour chaque concentration, une culture a été réalisée avec 6 puits.

Après 48 heures d'incubation, les cellules ont été fixées dans une solution de paraformaldéhyde (Sigma) à 2% de concentration finale dans le milieu de culture, puis les cellules ont été lavées 2 fois par un tampon phosphate (PBS, PanBiotech). Les sites non spécifiques ont été bloqués par une solution de PBS contenant 0,1% de saponine (Sigma), 5% de sérum de chèvre (SC ; PanBiotech) et 1% de BSA (*Bovin Serum Albumin* ; Panbiotech), pendant 15 minutes à température ambiante. Les cellules ont, par la suite, été incubées en présence d'un anticorps monoclonal de souris anti β-tubuline (Sigma) au 1/400ème dans du PBS contenant 5% de SC et 0,1% de saponine pendant 2 heures à 4°C. L'anticorps anti β-tubuline marque les corps cellulaires et les prolongements des neurones sensitifs humains. Cet anticorps a été révélé par un Alexa Fluor 488 chèvre anti-souris IgG (Molecular Probe) au 1/400ème dans une solution de PBS contenant 5% de SC, 0,1% de saponine et 1% de BSA pendant 1 heure à température ambiante et à l'abri de la lumière. Les noyaux ont été marqués par une solution de Hoechst (Sigma), un marqueur fluorescent nucléaire, au 1/10 000éme dans la même solution que l'anticorps secondaire.

Par puits de culture, 20 photographies ont été prises avec un microscope automatique (InCell 2000 ; GE Healthcare) au grossissement x20.

### 1.3. Evaluation de l'effet de l'HE d'immortelle sur la pousse neuritique

Dans une deuxième culture, après 24 heures de co-culture, le milieu a été changé par du milieu constitué de 2/3 de milieu de maturation des neurones sensitifs humains sans facteurs de croissance et 1/3 de milieu de croissance pour kératinocytes, en présence ou non du composé à tester à différentes concentrations ou de la molécule de référence constituée de NGF à 50ng/mL. Le NGF ou facteur de croissance neuronal, qui est naturellement présent dans l'épithélium, est connu pour stimuler l'allongement des neurites in vitro. Une culture a été réalisée avec 6 puits par concentration testée.

La culture a été maintenue pendant 6 jours et le milieu de culture contenant les composés de l'étude a été changé au bout de 3 jours.

Après 6 jours de culture, le milieu de culture a été éliminé et les cellules ont été fixées par une solution de paraformaldéhyde à 2% de concentration finale dans le milieu de culture, puis les cellules ont été lavées 2 fois avec du PBS. Les sites non spécifiques ont été bloqués par une solution de PBS contenant 0,1% de saponine, 5% de sérum de chèvre et 1% de BSA, pendant 15 minutes à température ambiante. Les cellules ont, par la suite, été incubées en présence d'un anticorps monoclonal de souris anti β-tubuline au 1/400ème dans du PBS contenant 5% de SC et 0,1% de saponine pendant 2 heures à 4°C. L'anticorps anti β-tubuline marque les corps cellulaires et les prolongements des neurones sensitifs humains. Cet anticorps a été révélé par un Alexa Fluor 488 chèvre anti-souris IgG au 1/400ème dans une solution de PBS contenant 5% de SC, 0,1% de saponine et 1% de BSA pendant 1 heure à température ambiante et à l'abri de la lumière. Les noyaux ont été marqués par une solution de Hoechst (Sigma), un marqueur fluorescent nucléaire, au 1/10 000éme dans la même solution que l'anticorps secondaire.

Par puits de culture, 20 photographies ont été prises avec le microscope automatique (InCell 2000 ; GE Healthcare) au grossissement x20 et une numération des corps cellulaires des neurones sensitifs a été effectuée. La longueur des prolongements a également été mesurée et normalisée par le nombre de corps cellulaires de neurones marqués. Les résultats obtenus pour les conditions de traitement par le composé à évaluer et le NGF ont été comparés au milieu témoin.

### 1.4. Etudes statistiques

Les études statistiques ont été faites par le test One Way ANOVA.

### 2. Résultats

### 2.1. Cytotoxicité de l'huile essentielle d'immortelle

L'huile essentielle d'Immortelle n'est pas toxique pour les neurones sensitifs humains aux concentrations évaluées.

### 2.2. Evaluation de l'effet de l'HE d'immortelle sur la pousse neuritique

L'huile essentielle d'Immortelle aux concentrations évaluées n'entraîne pas de modulation du nombre de neurones sensitifs humains. Ceci est en accord avec les résultats obtenus lors de l'étude de cytotoxicité.

Le NGF à 50ng/mL entraîne une augmentation de la longueur des prolongements des neurones sensitifs humains (+22,8 %, p<0.05). Ce résultat valide l'expérience. L'huile essentielle d'Immortelle entraine une augmentation significative de la longueur des prolongements des neurones sensitifs aux concentrations de 0,01%, 0,005% et 0,001% (augmentation respectivement de 35,8%, p<0.001, 30,8% p<0.001 et 40,3% p<0.001).

### 3. Conclusion

L'huile essentielle d'Immortelle augmente la densité d'innervation en augmentant la longueur des prolongements des neurones sensitifs humains en co-culture avec des kératinocytes.

### Exemple 2 : Composition cosmétique

La composition suivante a été préparée de manière classique pour l'homme de l'art, en mélangeant les ingrédients ci-dessous dans les proportions pondérales indiquées.

| | |
|---|---|
| Emulsionnants non ioniques | 6,00 % |
| Beurre de karité | 2,00 % |
| Epaississants de phase grasse | 6,50 % |
| Huiles | 8,25 % |
| Adénosine | 0,04 % |
| Glycérine | 6,00 % |
| Epaississants de phase aqueuse | 2,50 % |
| Huile essentielle d'immortelle | 0,02 % |
| Hydroxyde de sodium | 1,00 % |
| Conservateurs | qs |
| Parfum | qs |
| Eau | qsp 100,00 % |

## Revendications

1. Utilisation cosmétique non thérapeutique d'une huile essentielle d'immortelle de l'espèce *Helichrysum italicum,* appliquée topiquement sur la peau, pour lutter contre la diminution de la perception tactile cutanée due à l'âge.

2. Utilisation selon la revendication 1, **caractérisée en ce que** l'huile essentielle est appliquée sur la peau du visage et/ou des mains.

3. Utilisation selon l'une quelconque des revendications 1 et 2, **caractérisée en ce que** l'huile essentielle est appliquée sur la peau de personnes âgées.

4. Utilisation selon l'une quelconque des revendications 1 à 3, **caractérisée en ce que** l'huile essentielle est incluse dans une composition se présentant sous la forme d'une émulsion huile-dans-eau ou eau-dans-huile.

5. Utilisation selon la revendication 4, **caractérisée en ce que** la composition renferme de 0,001 à 5% en poids et préférentiellement de 0,01 à 2% en poids d'huile essentielle d'immortelle, par rapport au poids total de la composition.

6. Huile essentielle d'immortelle de l'espèce *Helichrysum italicum,* pour son utilisation topique dans un traitement visant à augmenter ou restaurer la perception tactile cutanée, par application sur la peau de personnes souffrant de neuropathies périphériques, de maladies neurodégénératives ou d'une destruction de terminaisons nerveuses consécutive à une plaie telle qu'une brûlure.

## Patentansprüche

1. Nicht-therapeutische kosmetische Verwendung eines ätherischen Öls aus Immortelle der Spezies *Helichrysum italicum,* das topisch auf die Haut angewendet wird, um die altersbedingte Verringerung der taktilen Wahrnehmung der Haut zu bekämpfen.

2. Verwendung nach Anspruch 1, **dadurch gekennzeichnet, dass** das ätherische Öl auf die Haut des Gesichts und/oder der Hände angewendet wird.

3. Verwendung nach einem der Ansprüche 1 und 2, **dadurch gekennzeichnet, dass** das ätherische Öl auf die Haut älterer Personen angewendet wird.

4. Verwendung nach einem der Ansprüche 1 bis 3, **dadurch gekennzeichnet, dass** das ätherische Öl in eine Zusammensetzung eingebracht ist, die in Form einer Ölin-Wasser- oder Wasser-in-Öl-Emulsion vorliegt.

5. Verwendung nach Anspruch 4, **dadurch gekennzeichnet, dass** die Zusammensetzung 0,001 bis 5 Gew.-% und vorzugsweise 0,01 bis 2 Gew.-% ätherisches Öl aus Immortelle, bezogen auf das Gesamtgewicht der Zusammensetzung, enthält.

6. Ätherisches Öl aus Immortelle der Spezies *Helichrysum italicum* für die topische Verwendung bei einer Behandlung zur Verbesserung oder Wiederherstellung der taktilen Wahrnehmung der Haut durch Anwenden auf die Haut von Personen, die an peripheren Neuropathien, neurodegenerativen Erkrankungen oder einer Zerstörung von Nervenendigungen infolge einer Wunde, wie einer Verbrennung, leiden.

## Claims

1. The non-therapeutic cosmetic use of an essential oil of everlasting flower of the species *Helichrysum italicum,* applied topically to the skin, for combating the decrease in skin touch perception due to age.

2. The use as claimed in claim 1, **characterized in that** the essential oil is applied to the skin of the face and/or of the hands.

3. The use as claimed in any one of claims 1 and 2, **characterized in that** the essential oil is applied to the skin of elderly individuals.

4. The use as claimed in any one of claims 1 to 3, **characterized in that** the essential oil is included in a composition which is in the form of an oil-in-water or water-in-oil emulsion.

5. The use as claimed in claim 4, **characterized in that** the composition contains from 0.001% to 5% by weight and preferentially from 0.01% to 2% by weight of essential oil of everlasting flower, relative to the total weight of the composition.

6. An essential oil of everlasting flower of the species *Helichrysum italicum,* for topical use thereof in a treatment intended to increase or restore skin touch perception, by application to the skin of individuals suffering from peripheral neuropathies, from neurodegenerative diseases or from a destruction of nerve endings subsequent to a wound such as a burn.
